(19) Europäisches Patentamt
European Patent Office
Office européen des brevets

(11) EP 2 837 644 A1

(12) **EUROPEAN PATENT APPLICATION**
published in accordance with Art. 153(4) EPC

(43) Date of publication:
**18.02.2015 Bulletin 2015/08**

(21) Application number: **13775207.7**

(22) Date of filing: **02.04.2013**

(51) Int Cl.:
***C08G 59/24*** *(2006.01)*

(86) International application number:
**PCT/JP2013/060069**

(87) International publication number:
**WO 2013/153988 (17.10.2013 Gazette 2013/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.04.2012 JP 2012091942**

(71) Applicant: **Daicel Corporation**
**Osaka-shi, Osaka 530-0001 (JP)**

(72) Inventors:
- **KITAO, Kyuhei**
  **Himeji-shi**
  **Hyogo 671-1283 (JP)**
- **TAKAI, Hideyuki**
  **Himeji-shi**
  **Hyogo 671-1283 (JP)**
- **SUGAHARA, Michihiro**
  **Himeji-shi**
  **Hyogo 671-1283 (JP)**

(74) Representative: **Grünecker, Kinkeldey,**
**Stockmair & Schwanhäusser**
**Leopoldstrasse 4**
**80802 München (DE)**

# (54) DIEPOXY COMPOUND AND METHOD FOR PRODUCING SAME

(57) There is provided a diepoxy compound that can be polymerized to form a cured product having thermal stability that has excellent heat resistance, and can maintain excellent mechanical properties even if exposed to a high temperature environment.

The saturated diepoxy compound of the present invention is represented by the following formula (1) and can be produced by hydrogenating the unsaturated bond of an unsaturated diepoxy compound represented by the following formula (2). In the following formulas, $R^1$ to $R^{20}$ are each an independent group and identically or differently represent a hydrogen atom, a methyl group, or an ethyl group.

EP 2 837 644 A1

[Formula 1]

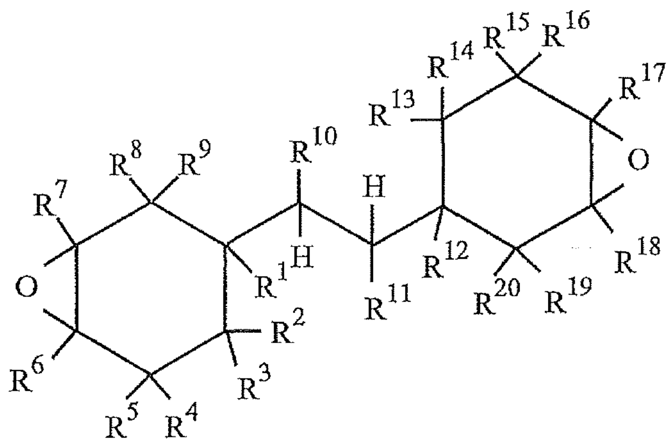

(1)

[Formula 2]

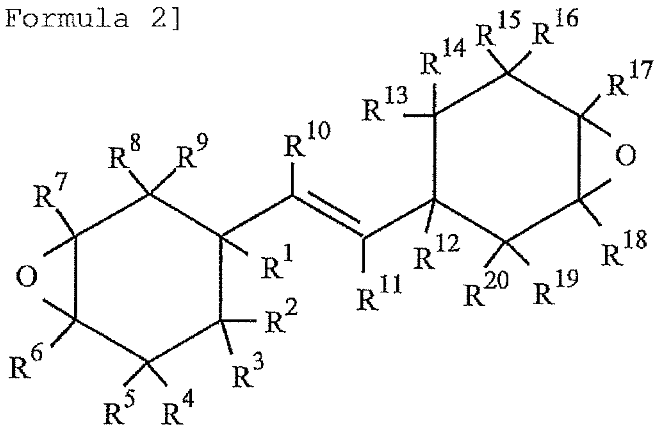

(2)

## Description

Technical Field

The present invention relates to a diepoxy compound that can be polymerized to be rapidly cured to form a cured product having excellent heat resistance, and a method for producing the same.

Background Art

It is known that epoxy compounds are polymerized to form cured products having excellent electrical characteristics, moisture resistance, heat resistance, and the like. Among them, diepoxy compounds are used in various fields including applications such as coating agents, inks, adhesives, sealants, sealing agents, resists, composite materials, transparent base materials, transparent films or sheets, optical materials (for example, optical lenses), insulating materials, stereolithographic materials, and electronic materials (for example, electronic paper, touch panels, solar cell substrates, optical waveguides, light guide plates, and holographic memories).
As diepoxy compounds, various types of them are currently commercially available, and examples of the diepoxy compounds include 3,4-epoxycyclohexylmethyl (3,4-epoxy)cyclohexanecarboxylate and 3,4,3',4'-diepoxybicyclohexyl. By polymerizing these diepoxy compounds with various curing agents or curing catalysts, cured products are obtained.
However, a problem of the above 3,4-epoxycyclohexylmethyl (3,4-epoxy)cyclohexanecarboxylate is that it has low oxirane oxygen concentration and a small number of crosslinking points, and therefore, the strength and heat resistance of the obtained cured product are low.
On the other hand, 3,4,3',4'-diepoxybicyclohexyl has higher oxirane oxygen concentration than 3,4-epoxycyclohexylmethyl (3,4-epoxy)cyclohexanecarboxylate, and therefore, the obtained cured product has better heat resistance but is not yet sufficiently satisfiable in that the mechanical properties (for example, elastic modulus) decrease greatly with temperature increase, and the thermal stability is low.

Citation List

Patent Literature

Patent Literature 1: Japanese Patent Laid-Open No. 63-264625

Patent Literature 2: Japanese Patent Laid-Open No. 63-012623

Patent Literature 3: Japanese Patent Laid-Open No. 59-011317

Patent Literature 4: Japanese Patent Laid-Open No. 2008-31424

Summary of Invention

Technical Problem

Accordingly, it is an object of the present invention to provide a diepoxy compound that is polymerized to form a cured product having thermal stability that has excellent heat resistance, and can maintain excellent mechanical properties even if exposed to a high temperature environment.
It is another object of the present invention to provide a method for producing the above diepoxy compound.

Solution to Problem

The present inventors have studied diligently in order to solve the above problems and as a result found that 3,4,3',4'-diepoxybicyclohexyl has a structure in which two alicyclic epoxy groups (= cyclic groups formed by linking two carbon atoms constituting an alicyclic ring and one oxygen atom to each other) are rigidly bonded by a single bond, and therefore, two alicyclic epoxy groups cannot always be present at positions suitable for a crosslinking reaction, and an alicyclic epoxy group occurs that cannot be involved in the crosslinking reaction, and although the oxirane oxygen concentration of the monomer is high, a cured product having sufficient heat resistance is not obtained. The present inventors have found that in a compound having a structure in which two alicyclic epoxy groups are flexibly bonded by

a hydrocarbon chain, the alicyclic epoxy groups can always be present at positions suitable for a crosslinking reaction, and the number of alicyclic epoxy groups that cannot be involved in the crosslinking reaction can be significantly reduced, and thus, it is possible to form a cured product having thermal stability that has extremely high heat resistance, and can maintain excellent mechanical properties even if exposed to a high temperature environment. The present invention has been completed based on these findings.

Specifically, the present invention provides a saturated diepoxy compound represented by the following formula (1):

[Formula 1]

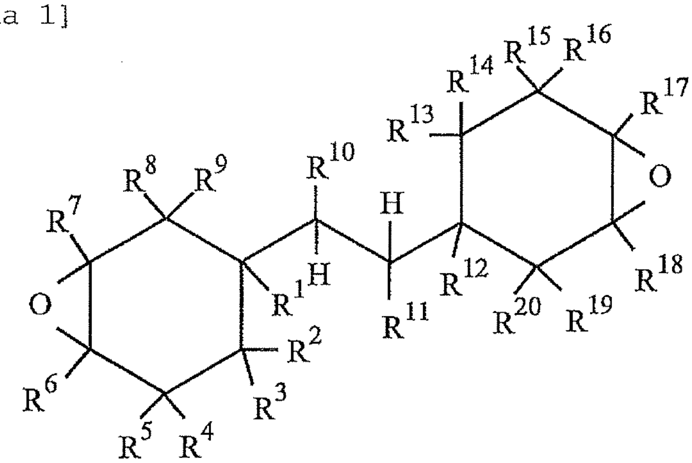

(1)

wherein $R^1$ to $R^{20}$ are each an independent group and identically or differently represent a hydrogen atom, a methyl group, or an ethyl group.

The present invention also provides a method for producing a saturated diepoxy compound, comprising hydrogenating an unsaturated bond of an unsaturated diepoxy compound represented by the following formula (2) :

[Formula 2]

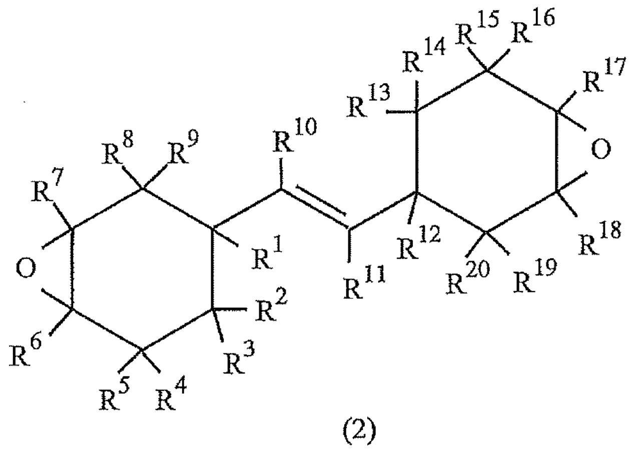

(2)

wherein $R^1$ to $R^{20}$ are each an independent group and identically or differently represent a hydrogen atom, a methyl group, or an ethyl group,
to obtain a saturated diepoxy compound represented by the following formula (1):

[Formula 3]

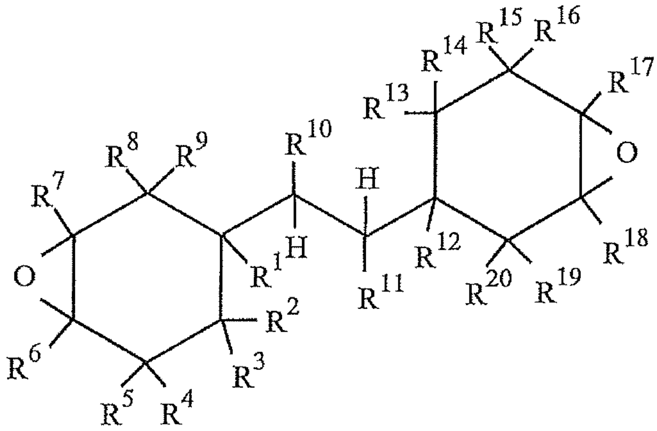

(1)

wherein $R^1$ to $R^{20}$ are as defined above.

It is preferred that the hydrogenation reaction is performed under a hydrogen atmosphere using a palladium compound as a catalyst.

The present invention further provides an unsaturated diepoxy compound represented by the following formula (2):

[Formula 4]

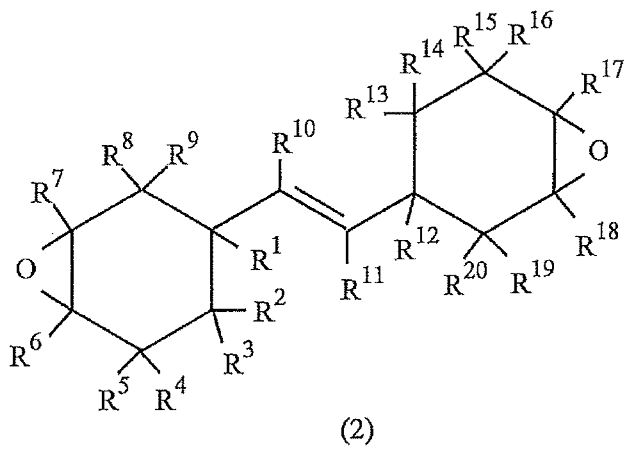

(2)

wherein $R^1$ to $R^{20}$ are each an independent group and identically or differently represent a hydrogen atom, a methyl group, or an ethyl group.

The present invention further provides a method for producing an unsaturated diepoxy compound, comprising subjecting an epoxy compound represented by the following formula (3):

[Formula 5]

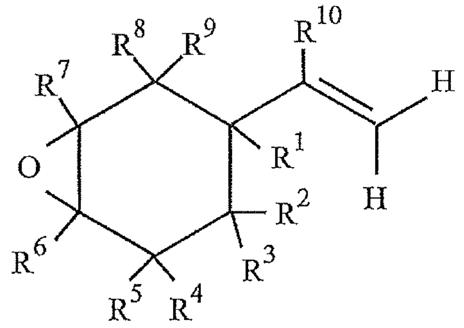

(3)

wherein $R^1$ to $R^{10}$ are each an independent group and identically or differently represent a hydrogen atom, a methyl group, or an ethyl group, and an epoxy compound represented by the following formula (3'):

[Formula 6]

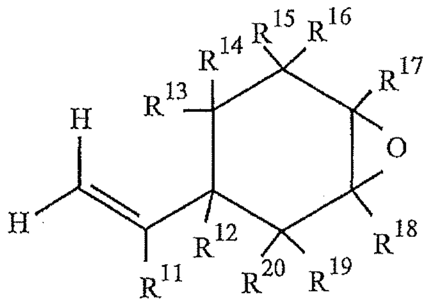

(3')

wherein $R^{11}$ to $R^{20}$ are each an independent group and identically or differently represent a hydrogen atom, a methyl group, or an ethyl group,
to a metathesis reaction to obtain an unsaturated diepoxy compound represented by the following formula (2):

[Formula 7]

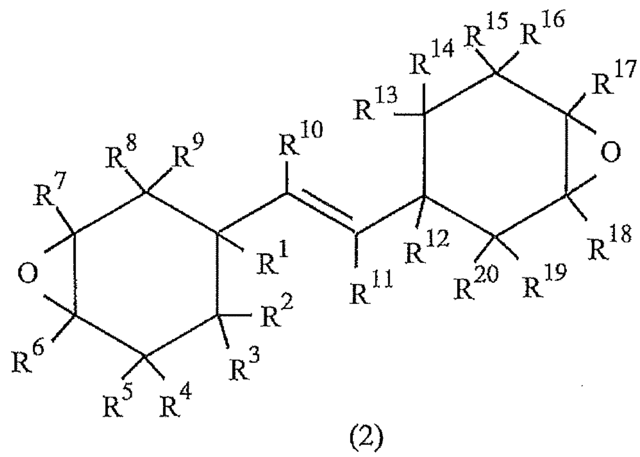

(2)

wherein $R^1$ to $R^{20}$ are as defined above.

The present invention also provides a method for producing an unsaturated diepoxy compound, comprising subjecting a compound represented by the following formula (4):

[Formula 8]

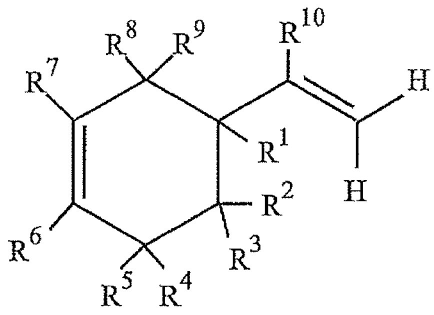

(4)

wherein $R^1$ to $R^{10}$ are each an independent group and identically or differently represent a hydrogen atom, a methyl group, or an ethyl group, and
a compound represented by the following formula (4'):

[Formula 9]

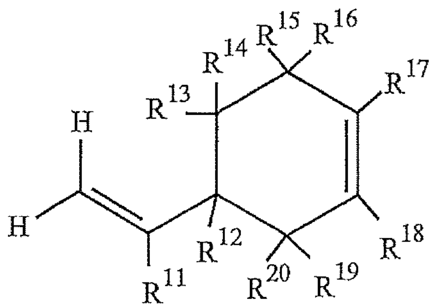

(4')

wherein $R^{11}$ to $R^{20}$ are each an independent group and identically or differently represent a hydrogen atom, a methyl group, or an ethyl group,
to a metathesis reaction to obtain a compound represented by the following formula (5):

[Formula 10]

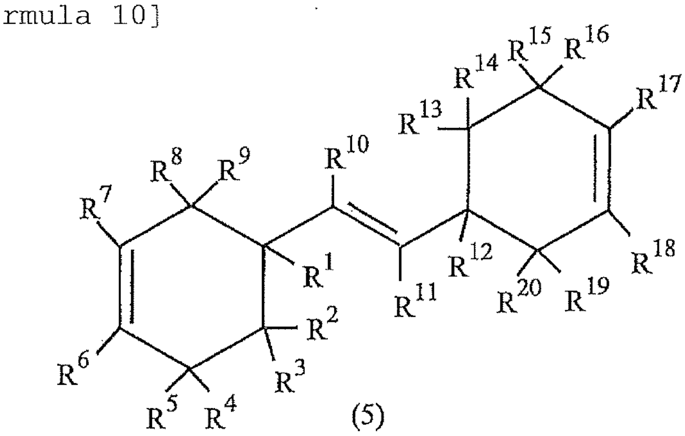

(5)

wherein $R^1$ to $R^{20}$ are as defined above, and epoxidizing the obtained compound represented by the formula (5) to obtain an unsaturated diepoxy compound represented by the following formula (2):

[Formula 11]

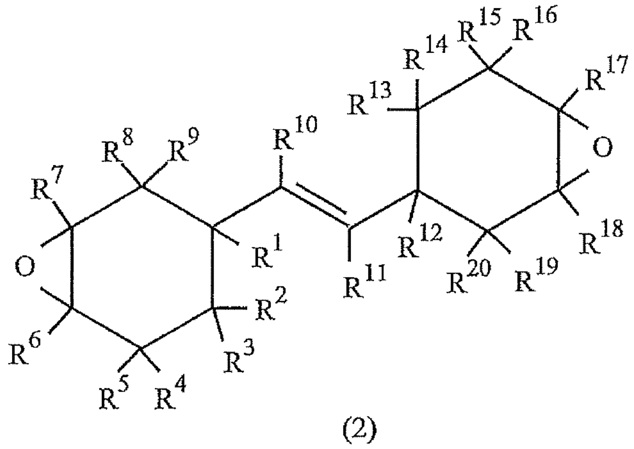

(2)

wherein $R^1$ to $R^{20}$ are as defined above.

It is preferred that a ruthenium complex is used as a catalyst in the metathesis reaction.

It is preferred that a peracid is used as an epoxidizing agent in the epoxidation reaction.

The present invention also provides a curable composition comprising the above saturated diepoxy compound.

The present invention further provides a cured product obtained by curing the above curable composition.

## Advantageous Effect of Invention

The saturated diepoxy compound represented by formula (1) in the present invention has a structure in which two alicyclic epoxy groups are flexibly bonded by a saturated hydrocarbon chain, and therefore, the alicyclic epoxy groups can move appropriately to positions suitable for a crosslinking reaction, and the number of alicyclic epoxy groups not involved in the crosslinking reaction can be reduced to an extremely small number. Therefore, it is possible to form a cured product in which the crosslinked structure can be densely formed and which has excellent heat resistance, and can maintain excellent mechanical properties even if exposed to a high temperature environment. The saturated diepoxy compound of the present invention can be preferably used in various fields including applications, for example, coating agents, inks, adhesives, sealants, sealing agents, resists, composite materials, transparent base materials, transparent films or sheets, optical materials (for example, optical lenses), insulating materials, stereolithographic materials, and electronic materials (for example, electronic paper, touch panels, solar cell substrates, optical waveguides, light guide plates, and holographic memories).

## Description of Embodiments

The saturated diepoxy compound of the present invention is a cationic curable compound represented by the following formula (1). In formula (1), $R^1$ to $R^{20}$ are each an independent group and identically or differently represent a hydrogen atom, a methyl group, or an ethyl group.

[Formula 12]

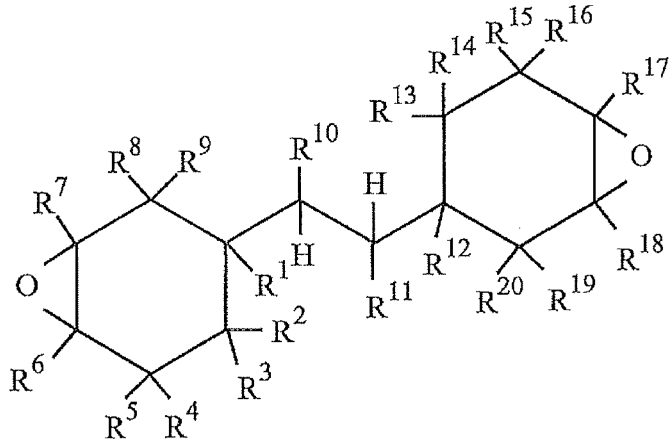

(1)

The saturated diepoxy compound represented by the above formula (1) can be produced, for example, by hydrogenating the unsaturated bond of an unsaturated diepoxy compound represented by the following formula (2) :

[Formula 13]

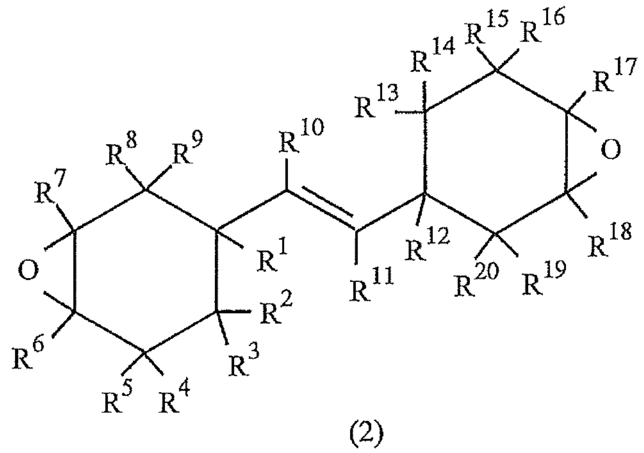

(2)

wherein $R^1$ to $R^{20}$ are as defined above.

The hydrogenation reaction is preferably performed in the presence of a catalyst. As the above catalyst, metal catalysts (catalysts comprising metal simple substances or metal compounds) effective for the hydrogenation reaction are preferably used. Examples of the metal catalysts include platinum catalysts, palladium catalysts, rhodium catalysts, iridium catalysts, ruthenium catalysts, and nickel catalysts. Among these, palladium catalysts are preferred, palladium carbon catalysts (comprising activated carbon as a support and having palladium(0) dispersed and supported on the surface of the support), palladium-fibroin composites, and the like are particularly preferred, and palladium carbon-ethylenediamine complexes and the like are most preferred.

The amount of the metal catalyst used (in terms of metal) is, for example, about 0.05 to 5 parts by weight, based on 100 parts by weight of the unsaturated diepoxy compound represented by formula (2). The upper limit of the amount of the metal catalyst used is preferably 2.5 parts by weight, particularly preferably 1 part by weight. The lower limit is preferably 0.1 parts by weight, particularly preferably 0.25 parts by weight. When the amount of the metal catalyst used is less than the above range, the yield of the saturated diepoxy compound represented by formula (1) tends to decrease. On the other hand, when the amount of the metal catalyst used is more than the above range, poor economy may result.

The hydrogenation reaction is preferably performed in the presence of a solvent. The solvent is not particularly limited as long as it does not inhibit the progress of the reaction, and examples of the solvent include alcohols (for example, methanol, ethanol, propanol, and isopropanol) and ethers (for example, diethyl ether and tetrahydrofuran

(THF)). The amount of the solvent used is, for example, about 100 to 3000 parts by weight, preferably 1000 to 2000 parts by weight, for example, based on 100 parts by weight of the unsaturated diepoxy compound represented by formula (2).

The reaction pressure is, for example, about normal pressure to 100 atmospheres (0.1 to 10 MPa), preferably about normal pressure to 10 atmospheres (0.1 to 1 MPa). The hydrogenation reaction can be performed in the presence of hydrogen (under a hydrogen atmosphere) or under a hydrogen flow. In addition to hydrogen, for example, inert gases such as nitrogen, argon, and helium may be present in the gas phase portion of the reaction system. In order to enhance gas-liquid contact, a hydrogen-containing gas may be bubbled into the reaction system liquid phase portion through a bubbling tube. The reaction temperature is, for example, about 10°C to 50°C. The reaction time is, for example, about 5 to 100 hours. In addition, the reaction can be performed by any method such as a batch type, a semi-batch type, or a continuous type.

The carbon-carbon double bond of the unsaturated diepoxy compound represented by formula (2) is hydrogenated by the above hydrogenation reaction to produce the corresponding saturated diepoxy compound represented by formula (1).

After the completion of the reaction, the reaction product can be separated and purified, for example, by separation and purification means such as filtration, concentration, distillation, extraction, crystallization, recrystallization, adsorption, and column chromatography and means combining these.

In addition, the unsaturated diepoxy compound represented by the above formula (2) can be produced by the method of the following 1 or 2. In the following formulas, $R^1$ to $R^{20}$ are each an independent group and identically or differently represent a hydrogen atom, a methyl group, or an ethyl group.

1. An epoxy compound represented by the following formula (3) and an epoxy compound represented by the following formula (3') are subjected to a metathesis reaction (particularly an olefin metathesis reaction).

[Formula 14]

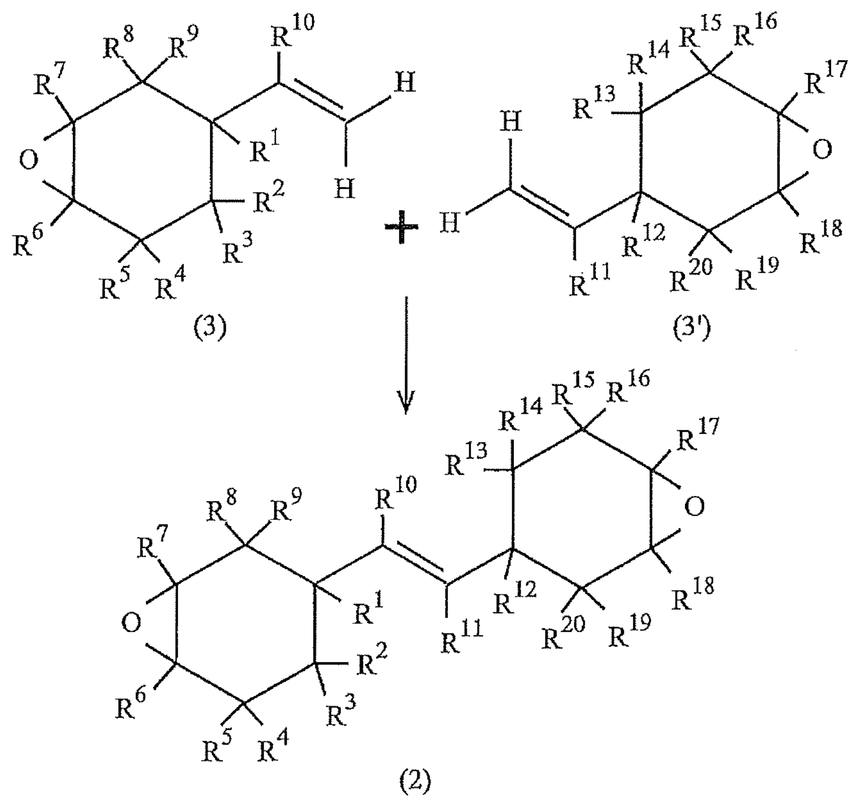

2. A compound represented by the following formula (4) and a compound represented by the following formula (4') are subjected to a metathesis reaction to obtain a compound represented by the following formula (5), and the obtained compound represented by the above formula (5) is epoxidized.

[Formula 15]

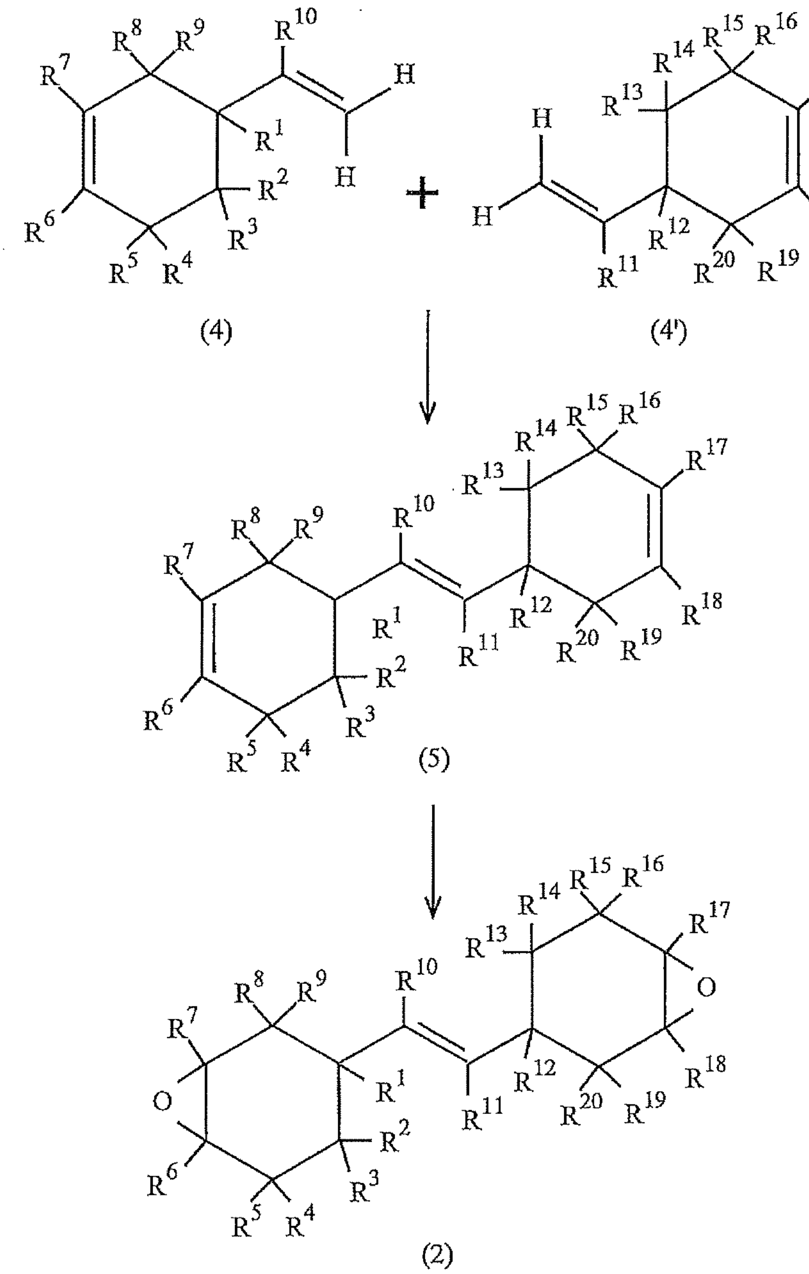

(4) (4') (5) (2)

The above metathesis reaction is preferably performed in the presence of a catalyst. As the above catalyst, for example, ruthenium complexes, tungsten compounds, molybdenum compounds, titanium compounds, and vanadium compounds are preferably used. In the present invention, for example, commercial products of ruthenium carbene complexes such as Dichloro-(3-phenyl-1H-inden-1-ylidene)bis(tricyclohexylphosphine)ruthenium(II) (trade name "Umicore M1"), Dichloro-(3-phenyl-1H-inden-1-ylidene)bis(isobutylphobane)ruthenium(II) (trade name "Umicore $M1_1$"), [1,3-Bis(2,4,6-trimethylphenyl)-2-imidazolidinylidene]dichloro-(3-phenyl-1H-inden-1-ylidene)(tricyclohexylphosphine)ruthenium(II) (trade name "Umicore M2"), [1,3-Bis(2,4,6-trimethylphenyl)-2-imidazolidinylidene]dichloro-(3-phenyl-1H-inden-1-ylidene)(pyridyl)ruthenium(II) (trade name "Umicore $M3_1$"), [1,3-Bis(2,4,6-trimethylphenyl)-2-imidazolidinylidene]-[2-[[(4-methylphenyl)imino]methyl]-4-nitro-phenolyl]chloro-[3-phenyl-indenylidene]ruthenium(II) (trade name "Umicore $M4_1$"), [1,3-Bis(2,4,6-trimethylphenyl)-2-imidazolidinylidene]-[2-[[(2-methylphenyl)imino]methyl]-phenolyl]chloro-(3-phenyl-indenylidene)ruthenium(II) (trade name "Umicore $M4_2$"), [1,3-bis(2,4,6-trimethylphenyl)-2-imidazolidinylidene]dichloro[2-(1-methylacetoxy)phenyl]methyleneruthenium(II) (trade name "Umicore $M5_1$", all the above products manufactured by Umicore), bis(tricyclohexylphosphine)benzylidyne ruthenium (IV) dichloride benzylidene-bis(tricyclohexylphosphine)dichlororuthenium (trade name "Grubbs Catalyst, 1st Generation"), (1,3-bis(2,4,6-trimethylphenyl)-2-imidazolidinylidene)dichloro(phenylmethylene) (tricyclohexylphosphine)ruthenium[1,3-bis-(2,4,6-trimethylphenyl)-2-imidazolidinylidene]dichloro(phenylmethylene)(tricyclohex ylphosphine)ruthenium benzylidene[1,3-bis(2,4,6-trimethylphenyl)-2-imidazolidinylidene]dichloro(tricyclohexylphosphine)ruthe nium (trade name "Grubbs Catalyst, 2nd Generation"), (dichloro-o-isopropoxyphenylmethylene)(tricyclohexylphosphine)rutheni um (II) (trade name "Hoveyda-Grubbs Catalyst 1st Generation"), (1,3-bis-(2,4,6-trimethylphenyl)-2-imidazolidinylidene)dichloro(o-isopropoxyphenylmethylene)ruthenium (trade name "Hoveyda-Grubbs Catalyst 2nd Generation", all the above products manufactured by Sigma-Aldrich Japan K.K.), Tricyclohexylphosphine[1,3-bis(2,4,6-trimethylphenyl)imidazol-2-ylidene][2-thienylmethylene]ruthenium(II) dichloride (trade name "catMETium RF 2"), Tricyclohexylphosphine[4,5-dimethyl-1,3-bis(2,4,6-trimethylphenyl)imidazol-2-ylidene][2-thienylmethylene]ruthenium(II) dichloride (trade name "catMETium RF 3"), Tricyclohexylphosphine[2,4-dihydro-2,4,5-triphenyl-3H-1,2,4-triazol-3-ylidene][2-thienylmethylene]ruthenium(II) dichloride (trade name "catMETium RF 4", all the above products manufactured by Evonik Industried AG) can be preferably used. In addition, combinations of compounds of transition metals of groups 4 to 8 of the periodic table such as tungsten chloride, tungsten oxide chloride, molybdenum chloride, titanium chloride, and vanadium chloride and organoaluminums such as triethylaluminum or organotins such as tetramethyltin can also be used, and in addition, molybdenum carbene complexes such as 2,6-Diisopropylphenylimido Neophylidenemolybdenum(VI) Bis(hexafluoro-t-butoxide) (manufactured by STREM) can also be used.

The amount of the catalyst used in the metathesis reaction is, for example, about 0.00001 to 0.01 moles, based on 1 mole of the epoxy compounds represented by formulas (3) and (3') [or the compounds represented by formulas (4) and (4')]. The upper limit of the amount of the catalyst used is preferably 0.005 moles, particularly preferably 0.003 moles. The lower limit is preferably 0.00002 moles, particularly preferably 0.00005 moles. When the amount of the catalyst used is less than the above range, the yield of the unsaturated diepoxy compound represented by formula (2) tends to decrease. On the other hand, when the amount of the catalyst used is more than the above range, poor economy may result.

The metathesis reaction may be performed in the presence of a solvent. The solvent is not particularly limited as long as it does not inhibit the progress of the reaction, and examples of the solvent include aliphatic hydrocarbons such as hexane and octane; aromatic hydrocarbons such as toluene and xylene; alicyclic hydrocarbons such as cyclohexane; halogenated hydrocarbons such as methylene chloride and 1,2-dichloroethane; esters such as ethyl acetate; ethers such as dioxane; and aprotic polar solvents such as N,N-dimethylformamide. The solvent can be used singly or in combinations of two or more.

The amount of the solvent used is, for example, about 0 to 2000 parts by weight, preferably 0 to 500 parts by weight, for example, based on 100 parts by weight of the total amount of the epoxy compounds represented by formulas (3) and (3') [or the total amount of the compounds represented by formulas (4) and (4')].

The reaction temperature can be appropriately selected according to the types of the reaction components and the catalyst, and the like, and is, for example, 10 to 100°C, preferably 20 to 80°C, and further preferably about 30 to 50°C. The reaction time is, for example, about 5 to 100 hours, preferably 12 to 60 hours. The reaction may be performed at normal pressure or under reduced pressure or increased pressure. The atmosphere of the reaction is not particularly limited unless the reaction is inhibited, and it may be any, for example, a nitrogen atmosphere or an argon atmosphere. In addition, the reaction can be performed by any method such as a batch type, a semi-batch type, or a continuous type.

After the completion of the reaction, the reaction product can be separated and purified, for example, by separation and purification means such as filtration, concentration, distillation, extraction, crystallization, recrystallization, adsorption, and column chromatography and means combining these.

When the epoxy compounds represented by formulas (3) and (3') are subjected to a metathesis reaction, the rearrangement of bonds occurs between the above two epoxy compounds, and the corresponding unsaturated diepoxy compound represented by formula (2) is produced. In addition, when the compounds represented by formulas (4) and (4') are subjected to a metathesis reaction, the rearrangement of bonds occurs between the above two epoxy compounds, and the corresponding compound represented by formula (5) is obtained.

By further epoxidizing the compound represented by the above formula (5) obtained by the above metathesis reaction, the corresponding unsaturated diepoxy compound represented by formula (2) is obtained.

The epoxidation reaction is preferably performed in the presence of an epoxidizing agent. Examples of the epoxidizing agent include peracids and hydrogen peroxide. In the present invention, among them, peracids are preferably used.

As the above peracids, for example, performic acid, peracetic acid, perbenzoic acid, meta-chloroperbenzoic acid, and trifluoroperacetic acid can be used. Among these, meta-chloroperbenzoic acid is preferably used as the epoxidizing agent in terms of easy availability.

The amount of the epoxidizing agent used is not particularly limited, can be appropriately adjusted according to the type of the epoxidizing agent used, the type of the compound represented by formula (5), and the like, and is, for example, about 1.6 to 2.4 moles, preferably 1.8 to 2.2 moles, for example, based on 1 mole of the compound represented

by formula (5).

The epoxidation reaction may be performed in the presence of a solvent. The solvent is not particularly limited as long as it does not inhibit the progress of the reaction, and examples of the solvent can include aromatic compounds such as toluene and benzene, aliphatic hydrocarbons such as hexane and cyclohexane, and esters such as ethyl acetate.

The reaction temperature in the epoxidation reaction is, for example, about 0 to 60°C, preferably 0 to 40°C, particularly preferably 0 to 20°C, and most preferably 0 to 10°C. When the reaction temperature is less than 0°C, the reaction may be slow. On the other hand, when the reaction temperature is more than 60°C, the decomposition of the epoxidizing agent may occur. The reaction can be performed, for example, by stirring a mixture of the compound represented by the above formula (5) and the epoxidizing agent for about 1 to 5 hours.

The epoxidation reaction can be completed, for example, by adding a reducing agent such as sodium sulfite, potassium sulfite, ammonium sulfite, sodium hydrogen sulfite, potassium hydrogen sulfite, ammonium hydrogen sulfite, sodium thiosulfate, or potassium thiosulfate to the reaction system to quench the above epoxidizing agent. After the completion of the reaction, the reaction product can be separated and purified, for example, by separation and purification means such as filtration, concentration, distillation, extraction, crystallization, recrystallization, adsorption, and column chromatography and means combining these.

The saturated diepoxy compound represented by formula (1) obtained by the above method has a structure in which two alicyclic epoxy groups are bonded by a saturated hydrocarbon chain, and therefore, the alicyclic epoxy groups can move appropriately to positions suitable for a crosslinking reaction, and the number of alicyclic epoxy groups not involved in the crosslinking reaction can be reduced to an extremely small number. Therefore, by cationic polymerization, it is possible to form a cured product having excellent thermal stability in which the crosslinked structure can be densely formed and which has excellent heat resistance, and can maintain excellent mechanical properties even if exposed to a high temperature environment.

In addition, in the saturated diepoxy compound represented by formula (1), as an industrial product, obtained by the above method, the content of positional isomers is extremely low, and the content of positional isomers is, for example, not more than 5%, preferably not more than 1%.

The curable composition of the present invention comprises the saturated diepoxy compound represented by the above formula (1). The content of the saturated diepoxy compound represented by the above formula (1) is, for example, not less than 20% by weight, preferably 20 to 99.5% by weight, and particularly preferably 25 to 95% by weight, based on the total amount of the curable composition (100% by weight). When the content of the saturated diepoxy compound represented by formula (1) is less than the above range, the heat resistance and thermal stability of the obtained cured product tend to decrease.

The curable composition of the present invention may contain another cationic curable compound (for example, an epoxy compound other than the saturated diepoxy compound represented by the above formula (1)) in addition to the saturated diepoxy compound represented by the above formula (1). In addition, the curable composition of the present invention may contain additives in addition to the cationic curable compounds. In the present invention, particularly, a cationic polymerization initiator is preferably contained. The cationic polymerization initiator includes a photo-cationic polymerization initiator and a thermal cationic polymerization initiator.

The photo-cationic polymerization initiator is a compound that generates a cationic species by being irradiated with active energy rays to initiate the cationic polymerization of an epoxy compound. In the present invention, a photo-acid-generator having the function of generating an acid by ultraviolet irradiation and initiating cationic polymerization by the generated acid is preferably used.

Examples of the above photo-acid-generator can include sulfonium salts (particularly triarylsulfonium salts) such as triarylsulfonium hexafluorophosphates (for example, p-phenylthiophenyldiphenylsulfonium hexafluorophosphate) and triarylsulfonium hexafluoroantimonates; iodonium salts such as diaryliodonium hexafluorophosphates, diaryliodonium hexafluoroantimonates, bis(dodecylphenyl)iodonium tetrakis(pentafluorophenyl)borate, and iodonium [4-(4-methylphenyl-2-methylpropyl)phenyl]hexafluorophosphate; phosphonium salts such as tetrafluorophosphonium hexafluorophosphate; and pyridinium salts such as N-hexylpyridinium tetrafluoroborate. These can be used singly or in combinations of two or more.

In the present invention, for example, commercial products such as the trade names "CPI-100P" and "CPI-101A" (manufactured by San-Apro Ltd.) may be used.

The amount of the photo-acid-generator used is, for example, about 0.05 to 10 parts by weight, preferably 0.1 to 8 parts by weight, based on 100 parts by weight of the saturated diepoxy compound represented by formula (1). When the amount of the photo-acid-generator used is less than the above range, the curing may be insufficient, and a long time may be required for the curing. On the other hand, when the amount of the photo-acid-generator used is more than the above range, the physical properties of the obtained cured product may decrease.

The thermal cationic polymerization initiator is a compound that generates a cationic species by heating to initiate the cationic polymerization of an epoxy compound, and examples of the thermal cationic polymerization initiator can include aromatic sulfonium salts and aromatic iodonium salts.

In the present invention, for example, commercial products such as the trade names "SI-60L," "SI-80L," and "SI-100L" (manufactured by SANSHIN CHEMICAL INDUSTRY CO., LTD.) and the trade names "CP-66" and "CP-77" (manufactured by ADEKA) may be used.

The amount of the thermal cationic polymerization initiator used is, for example, about 0.01 to 10 parts by weight, preferably 0.05 to 8 parts by weight, and most preferably 0.1 to 3 parts by weight, based on 100 parts by weight of the saturated diepoxy compound represented by formula (1). When the amount of the thermal cationic polymerization initiator used is less than the above range, the curing may be insufficient, and a long time may be required for the curing. On the other hand, when the amount of the thermal cationic polymerization initiator used is more than the above range, the physical properties of the obtained cured product may decrease.

As the cationic polymerization initiator, a combination of a chelate compound of a metal such as aluminum or titanium and a beta-diketone and a compound having a silanol group or bisphenol S (hereinafter sometimes referred to as a "chelating agent composition") can also be used in addition to the photo-acid-generator and the thermal cationic polymerization initiator. Examples of the beta-diketone coordinated to the metal such as aluminum or titanium include acetylacetone and acetoacetates. In the present invention, for example, commercial products such as the trade name "ALCH-TR" (manufactured by Kawaken Fine Chemicals Co., Ltd.) and the trade name "DAICAT EX-1" (manufactured by Daicel Corporation) may be used.

The amount of the chelating agent composition used is, for example, about 0.1 to 20 parts by weight, preferably 0.3 to 15 parts by weight, based on 100 parts by weight of the saturated diepoxy compound represented by formula (1). When the amount of the chelating agent composition used is less than the above range, the curing may be insufficient, and a long time may be required for the curing. On the other hand, when the amount of the chelating agent composition used is more than the above range, the physical properties of the obtained cured product may decrease.

As the additives, in addition to the cationic polymerization initiator, other additives, for example, a curing agent, a curing aid, an organosiloxane compound, metal oxide particles, rubber particles, a silicone-based or fluorine-based antifoaming agent, a silane coupling agent, a filler, a plasticizer, a leveling agent, an antistatic agent, a release agent, a flame retardant, a colorant, an antioxidant, an ultraviolet absorbing agent, an ion adsorbent, and a pigment can be further blended in the curable composition of the present invention as required. The amount of the other additives blended (when two or more are blended, their total amount) is, for example, not more than 5% by weight, based on the entire curable composition.

The curable composition of the present invention is prepared, for example, by blending the saturated diepoxy compound represented by the above formula (1), and additives and the like as required, and stirring and mixing them under vacuum as required while eliminating bubbles. The temperature in stirring and mixing is, for example, about 10 to 50°C. For the stirring and mixing, known apparatuses (for example, rotation-revolution type mixers, single-screw or multi-screw extruders, planetary mixers, kneaders, and dissolvers) can be used.

The curable composition prepared by the above method can be, for example, molded by a conventionally known molding method (for example, a casting method or an injection molding method) or applied by a conventionally known application method (for example, roll coat coating, spray coating, brush coating, bar coat coating, roller coating, silk screen printing, or spin coating) and then heated and/or irradiated with active energy rays to promote a curing reaction (cationic polymerization reaction) to form a cured product.

For the heating conditions, the heating temperature is about 50 to 200°C (preferably about 55 to 100°C, particularly preferably 60 to 80°C), and the heating time is about 0.5 to 5 hours (preferably 1 to 3 hours). Further, for example, heat treatment may be performed at a temperature of about 50 to 200°C (preferably 100 to 200°C) for about 0.5 to 5 hours (post-baking treatment), as required. By performing the post-baking treatment, the polymerization reaction proceeds further, and a cured product having better heat resistance can be formed.

For the active energy ray irradiation conditions, ultraviolet rays having a wavelength of about 250 to 400 nm are preferably used, and examples of the ultraviolet irradiation source can include high pressure mercury lamps, ultrahigh pressure mercury lamps, xenon lamps, carbon arcs, metal halide lamps, and sunlight. The amount of irradiation is, for example, about 0.1 to 20 $J/cm^2$. In addition, after the ultraviolet irradiation, for example, heat treatment may be performed at a temperature of about 50 to 200°C (preferably 100 to 200°C) for about 0.5 to 5 hours (post-baking treatment), as required. By performing the post-baking treatment, the polymerization reaction proceeds further, and a cured product having better heat resistance can be formed.

The above curing reaction may be performed under normal pressure, or under reduced pressure or under increased pressure. In addition, the reaction atmosphere of the curing reaction is not particularly limited unless the curing reaction is inhibited, and it may be any, for example, an air atmosphere, a nitrogen atmosphere, or an argon atmosphere.

The cured product of the present invention obtained by the above method has extremely excellent heat resistance, and the storage modulus (E') at 290°C is, for example, not less than $1.5 \times 10^8$ Pa, preferably not less than $5 \times 10^8$ Pa. In addition, the loss modulus (E'') at 290°C is, for example, not less than $1 \times 10^7$ Pa, preferably not less than $2 \times 10^7$ Pa.

Further, the cured product of the present invention obtained by the above method has excellent thermal stability,

and the changes in storage modulus (E') and loss modulus (E'') accompanying temperature change are extremely small, and the storage modulus (E') retention rate obtained by the following formula is, for example, not less than 60%, preferably not less than 65%. In addition, the loss modulus (E'') retention rate obtained by the following formula is, for example, not less than 45%, preferably not less than 50%.

storage modulus (E') retention rate (%) = [storage modulus (E') at 290°C]/[storage modulus (E') at 40°C] × 100

loss modulus (E'') retention rate (%) = [loss modulus (E'') at 290°C]/[loss modulus (E'') at 40°C] × 100

The curable composition comprising the saturated diepoxy compound represented by formula (1) in the present invention can form the above cured product having excellent heat resistance and thermal stability and therefore is used in various fields including applications such as coating agents, inks, adhesives, sealants, sealing agents, resists, composite materials, transparent base materials, transparent films or sheets, optical materials (for example, optical lenses), insulating materials, stereolithographic materials, and electronic materials (for example, electronic paper, touch panels, solar cell substrates, optical waveguides, light guide plates, and holographic memories).

Examples

The present invention will be more specifically described below by Examples, but the present invention is not limited by these Examples.

Example 1 (Synthesis of Epoxy Compound (I))

Under a nitrogen atmosphere, 1.0 g (corresponding to 0.0025 moles based on 1 mole of the following "CEL2000") of [1,3-Bis(2,4,6-trimethylphenyl)-2-imidazolidinylidene]dichloro-(3-phenyl-1H-inden-1-ylidene)(tricyclohexylphosphine)ruthenium(II) (trade name "Umicore M2," manufactured by Umicore) was dissolved in 53.3 g of toluene (Super Dehydrated, manufactured by Wako Pure Chemical Industries, Ltd.), and the solution was charged into a 200 mL three-necked flask.

While nitrogen was bubbled into the gas phase portion, 52.3 g of 1,2-epoxy-4-vinylcyclohexane (trade name "CEL2000," manufactured by Daicel Corporation) was charged by a syringe, and then, the mixture was stirred at 40°C for 48 hours. The reaction liquid was concentrated, and the obtained concentrated residue was purified by silica gel column chromatography to obtain 11.2 g of an epoxy compound represented by the following formula (I) (epoxy compound (I)) as a brown solid. The yield based on CEL2000 was 24.1%.

In $^1$H-NMR, it was confirmed that two types of peaks over δ4.8-5.8 corresponding to the olefin site of CEL2000 decreased to one type.

$^1$H-NMR(500MHz,$CDCl_3$,TMS standard) δ5.2-5.0(m,2H),3.1-3.0(m,4H),2.2-0.9(m,14H)

The oxirane oxygen concentration obtained by titration using an acetic acid solution of hydrogen bromide was 14.3% by weight, which was 99% of the theoretical value (14.5% by weight). In addition, using a differential thermal and thermogravimetric simultaneous measurement apparatus (TG/DTA) (trade name "EXSTAR TG/DTA6200," manufactured by SII NanoTechnology Inc.), the temperature was increased from 30°C to 400°C at 10°C/min while 200 mL/min of nitrogen was flowed, and the top temperature of endothermic peaks accompanying melting observed in this measurement was 50°C.

[Formula 16]

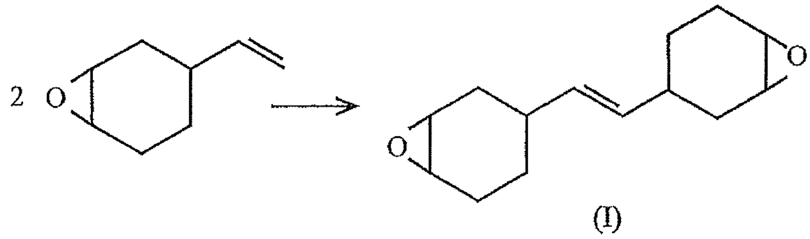

(I)

Example 2 (Synthesis of Epoxy Compound (II))

2.0 g (Pd: 0.1 g) of a 5% palladium carbon-ethylenediamine complex (5% Pd/C(en), manufactured by Wako Pure Chemical Industries, Ltd.) as a catalyst, 20.0 g of the epoxy compound (I) obtained in Example 1, and 363 g of THF were charged into a 1000 mL three-necked flask and then stirred under a hydrogen atmosphere at 30°C for 50 hours. The catalyst was removed by filtration, the obtained liquid was concentrated, and the obtained concentrated residue was purified by silica gel column chromatography to obtain 12.7 g of a pale yellow transparent liquid comprising an epoxy compound represented by the following formula (II) (epoxy compound (II)). The yield based on the epoxy compound (I) was 63%.

In $^1$H-NMR, the disappearance of peaks at δ5.2-5.0 corresponding to the double bond of the epoxy compound (I) was confirmed.

$^1$H-NMR(500MHz,$CDCl_3$,TMS standard) δ3.2-3.1(m,4H),2.2-0.8(m,18H)

The oxirane oxygen concentration obtained by titration using an acetic acid solution of hydrogen bromide was 14.3% by weight, which was 99% of the theoretical value (14.4% by weight).

[Formula 17]

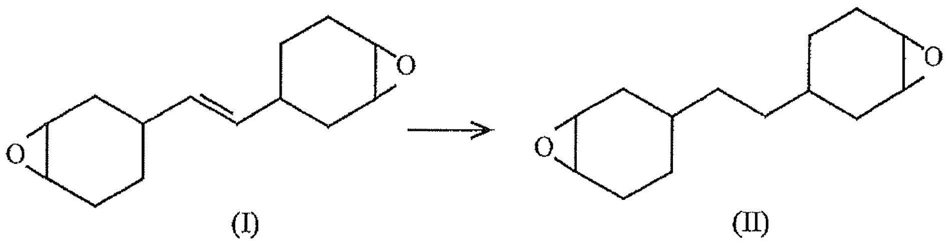

(I) (II)

Example 3 (Synthesis of Olefin Compound (III))

Under a nitrogen atmosphere, 0.08 g (corresponding to 0.0001 moles based on 1 mole of 4-vinyl-1-cyclohexene) of [1,3-Bis(2,4,6-trimethylphenyl)-2-imidazolidinylidene]dichloro-(3-phenyl-1H-inden-1-ylidene)(tricyclohexylphosphine)ruthenium(II) (trade name "Umicore M2," manufactured by Umicore) was dissolved in 90.0 g of toluene (Super Dehydrated, manufactured by Wako Pure Chemical Industries, Ltd.)), and the solution was charged into a 300 mL three-necked flask.

While nitrogen was bubbled into the gas phase portion, 89.5 g of 4-vinyl-l-cyclohexene was charged by a syringe, and then, the mixture was stirred at 40°C for 24 hours. The reaction liquid was concentrated, and the obtained concentrated residue was purified under reduced pressure (0.9 kPa) by simple distillation to obtain 37.1 g of an olefin compound represented by the following formula (III) (olefin compound (III)) as a fraction at 125 to 126°C. The yield based on 4-vinyl-1-cyclohexene was 47.4%.

In $^1$H-NMR, the disappearance of peaks of protons seen at δ5.1-4.9 corresponding to the terminal olefin of 4-vinyl-1-cyclohexene was confirmed.

$^1$H-NMR(500MHz,$CDCl_3$,TMS standard) δ5.7-5.6(m,4H),5.5-5.2(m,4H),2.3-1.3(m,14H)

[Formula 18]

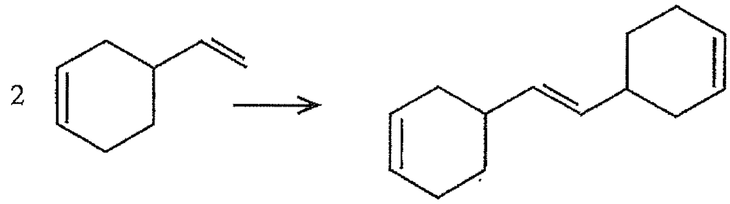

(III)

Example 4 (Synthesis of Epoxy Compound (I) from Olefin Compound (III))

1.0 g of the olefin compound (III) obtained in Example 3 was dissolved in 10 g of ethyl acetate. While the liquid temperature was kept at 0 to 5°C by icing, 2.6 g of hydrous meta-chloroperbenzoic acid (purity 70%) (corresponding to 2.0 moles based on 1 mole of the olefin compound (III)) was added over 20 minutes, and the mixture was stirred at 0 to 5°C for 2 hours. 17 g of a 10% by weight aqueous solution of sodium thiosulfate was added to the obtained reaction liquid, and the mixture was stirred for 30 minutes, and then, 10 g of toluene was added for separation, and the aqueous layer was subjected to extraction treatment with 10 g of toluene again.

The obtained organic layers were mixed and washed twice with 23 g of a 7% by weight aqueous solution of sodium hydrogen carbonate and twice with 20 g of water, and the organic layer was concentrated. 0.21 g of an epoxy compound represented by the following formula (I) (epoxy compound (I)) in 1.08 g of the obtained concentrated residue was quantified by a gas chromatography internal standard method. The yield based on the olefin compound (III) was 18%.

[Formula 19]

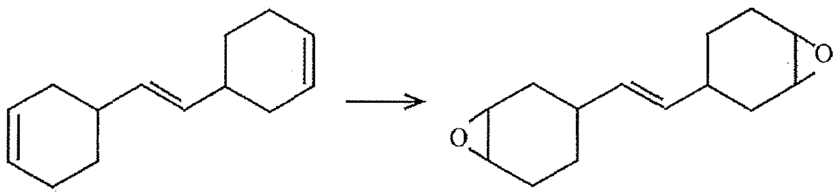

(III) (I)

Example 5

0.6 parts by weight of an aromatic sulfonium salt (trade name "SI-100L," manufactured by SANSHIN CHEMICAL INDUSTRY CO., LTD.) as a thermal cationic polymerization initiator was added to 100 parts by weight of the epoxy compound (II) to prepare a curable composition (1), and the curable composition (1) was cured at 65°C for 2 hours and then further cured at 150°C for 1.5 hours to obtain a transparent cured product (1).

Comparative Example 1

A curable composition (2) was prepared and a cured product (2) was obtained as in Example 5 except that 3,4-epoxycyclohexylmethyl (3,4-epoxy)cyclohexanecarboxylate represented by the following formula (trade name "CEL2021P," manufactured by Daicel Corporation) was used instead of the epoxy compound (II).

[Formula 20]

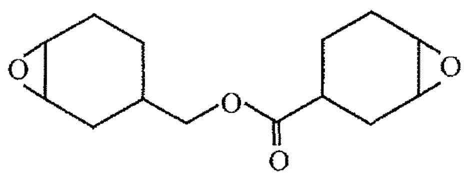

Comparative Example 2

A curable composition (3) was prepared and a cured product (3) was obtained as in Example 5 except that 3,4,3',4'-diepoxybicyclohexyl was used instead of the epoxy compound (II).

For the cured products (1) to (3) obtained in Example 5 and Comparative Examples 1 and 2, using a solid viscosity and elasticity measuring apparatus (trade name "RSA-III," manufactured by TA instrument), the storage modulus (E') and the loss modulus (E'') were measured in a tensile mode at a forced vibration frequency of 10 Hz under a nitrogen atmosphere while the temperature was increased from 10°C to 300°C at 5°C/min, and the temperature of a peak observed in a tan δ (E''/E') curve was calculated. The above peak temperature is used instead of glass transition temperature.

In addition, as indicators of thermal stability, the storage modulus retention rate (%) [storage modulus (E') at 290°C/storage modulus (E') at 40°C × 100] and the loss modulus retention rate (%) [loss modulus (E'') at 290°C/loss modulus (E'') at 40°C × 100] were calculated.

The above results are shown together in the following table. [Table 1]

Table 1

| | Example 5 | Comparative Example 1 | Comparative Example 2 |
|---|---|---|---|
| | Cured product (1) | Cured product (2) | Cured product (3) |
| Storage modulus at 290°C (Pa) | $7.71 \times 10^8$ | $1.41 \times 10^8$ | $1.46 \times 10^9$ |
| Storage modulus retention rate (%) | 71 | 7 | 57 |
| Loss modulus at 290°C (Pa) | $2.37 \times 10^7$ | $9.53 \times 10^5$ | $3.21 \times 10^7$ |
| Loss modulus retention rate (%) | 59 | 4 | 43 |
| tan δ peak temperature (°C) | No clear peak was seen | 243 | No clear peak was seen |

Industrial Applicability

The saturated diepoxy compound represented by formula (1) in the present invention can be polymerized to form a cured product in which the crosslinked structure can be densely formed and which has excellent heat resistance, and can maintain excellent mechanical properties even if exposed to a high temperature environment. Therefore, the saturated diepoxy compound represented by formula (1) in the present invention can be preferably used in various fields of, for example, coating agents, inks, adhesives, sealants, sealing agents, resists, composite materials, transparent base materials, transparent films or sheets, optical materials (for example, optical lenses), insulating materials, stereolithographic materials, and electronic materials (for example, electronic paper, touch panels, solar cell substrates, optical waveguides, light guide plates, and holographic memories).

## Claims

1. A saturated diepoxy compound represented by the following formula (1):

[Formula 1]

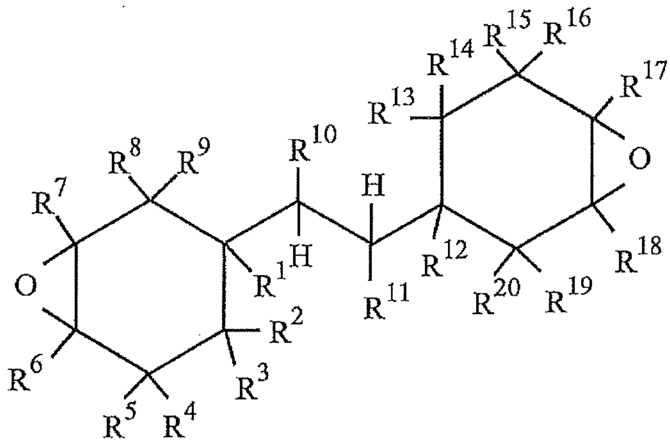

(1)

wherein $R^1$ to $R^{20}$ are each an independent group and identically or differently represent a hydrogen atom, a methyl group, or an ethyl group.

2. A method for producing a saturated diepoxy compound, comprising hydrogenating an unsaturated bond of an unsaturated diepoxy compound represented by the following formula (2):

[Formula 2]

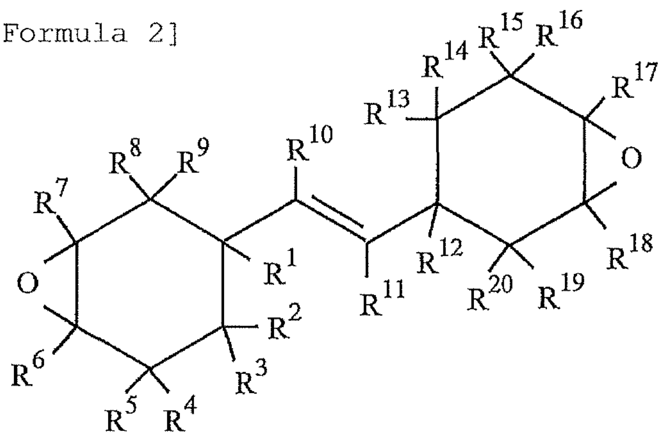

(2)

wherein $R^1$ to $R^{20}$ are each an independent group and identically or differently represent a hydrogen atom, a methyl group, or an ethyl group,
to obtain a saturated diepoxy compound represented by the following formula (1):

[Formula 3]

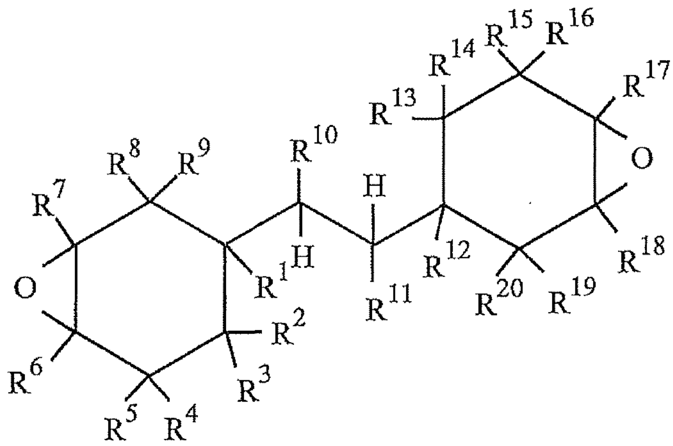

(1)

wherein $R^1$ to $R^{20}$ are as defined above.

**3.** The method for producing a saturated diepoxy compound according to claim 2, wherein a hydrogenation reaction is performed under a hydrogen atmosphere using a palladium compound as a catalyst.

**4.** An unsaturated diepoxy compound represented by the following formula (2):

[Formula 4]

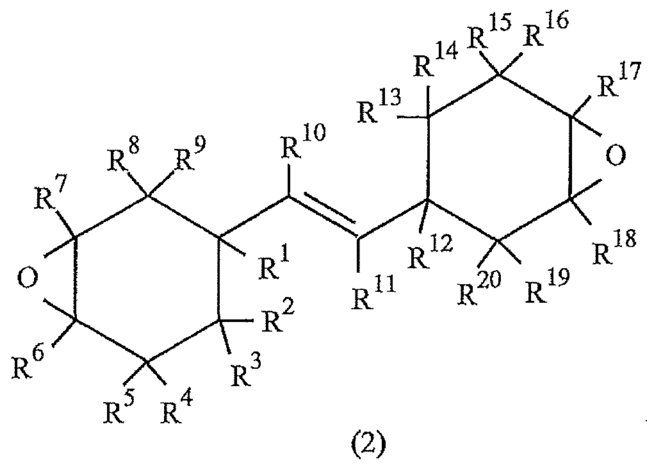

(2)

wherein $R^1$ to $R^{20}$ are each an independent group and identically or differently represent a hydrogen atom, a methyl group, or an ethyl group.

**5.** A method for producing an unsaturated diepoxy compound, comprising subjecting an epoxy compound represented by the following formula (3):

[Formula 5]

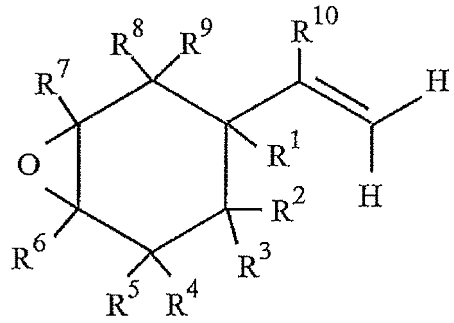

(3)

wherein $R^1$ to $R^{10}$ are each an independent group and identically or differently represent a hydrogen atom, a methyl group, or an ethyl group, and
an epoxy compound represented by the following formula (3'):

[Formula 6]

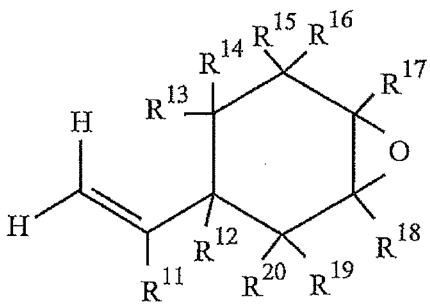

(3')

wherein $R^{11}$ to $R^{20}$ are each an independent group and identically or differently represent a hydrogen atom, a methyl group, or an ethyl group,
to a metathesis reaction to obtain an unsaturated diepoxy compound represented by the following formula (2):

[Formula 7]

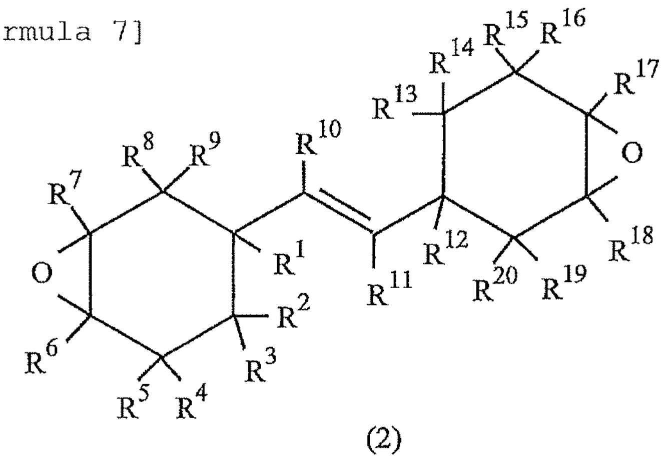

(2)

wherein $R^1$ to $R^{20}$ are as defined above.

**6.** A method for producing an unsaturated diepoxy compound, comprising subjecting a compound represented by the following formula (4):

[Formula 8]

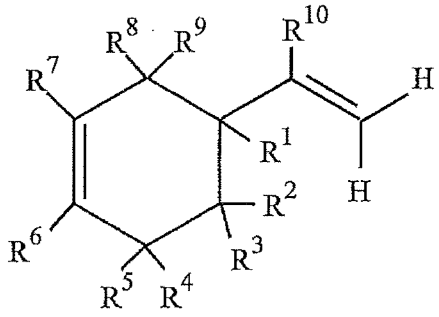

(4)

wherein $R^1$ to $R^{10}$ are each an independent group and identically or differently represent a hydrogen atom, a methyl group, or an ethyl group, and
a compound represented by the following formula (4'):

[Formula 9]

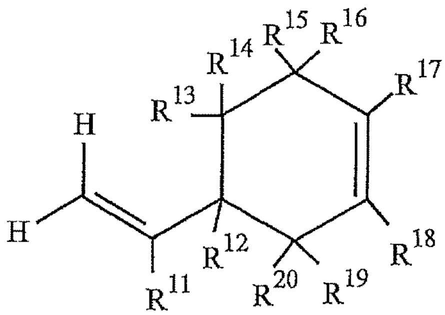

(4')

wherein $R^{11}$ to $R^{20}$ are each an independent group and identically or differently represent a hydrogen atom, a methyl group, or an ethyl group,
to a metathesis reaction to obtain a compound represented by the following formula (5):

[Formula 10]

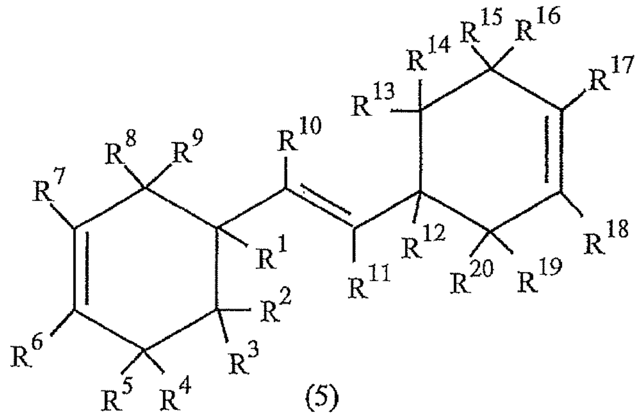

(5)

wherein $R^1$ to $R^{20}$ are as defined above, and epoxidizing the obtained compound represented by the formula (5) to obtain an unsaturated diepoxy compound represented by the following formula (2):

[Formula 11]

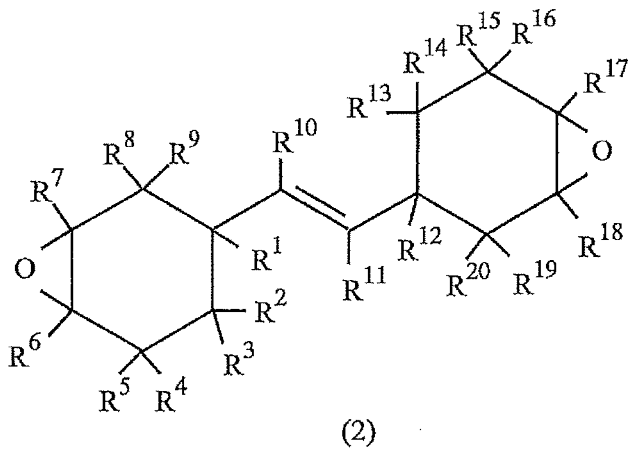

(2)

wherein $R^1$ to $R^{20}$ are as defined above.

**7.** The method for producing an unsaturated diepoxy compound according to claim 5 or 6, wherein a ruthenium complex is used as a catalyst in the metathesis reaction.

**8.** The method for producing an unsaturated diepoxy compound according to claim 6 or 7, wherein a peracid is used as an epoxidizing agent in an epoxidation reaction.

**9.** A curable composition comprising the saturated diepoxy compound according to claim 1.

**10.** A cured product obtained by curing the curable composition according to claim 9.

**INTERNATIONAL SEARCH REPORT**

International application No.
PCT/JP2013/060069

A. CLASSIFICATION OF SUBJECT MATTER
*C08G59/24*(2006.01)i

According to International Patent Classification (IPC) or to both national classification and IPC

B. FIELDS SEARCHED

Minimum documentation searched (classification system followed by classification symbols)
C08G59/24

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2011-168650 A (Daicel Chemical Industries, Ltd.), 01 September 2011 (01.09.2011), the entire specification & CN 102190863 A & KR 10-2011-0095172 A & TW 201136977 A | 1-10 |
| A | JP 2008-189699 A (Daicel Chemical Industries, Ltd.), 21 August 2008 (21.08.2008), the entire specification (Family: none) | 1-10 |
| A | JP 2012-1690 A (ADEKA Corp.), 05 January 2012 (05.01.2012), the entire specification (Family: none) | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 April, 2013 (25.04.13) | 14 May, 2013 (14.05.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 63264625 A
- JP 63012623 A
- JP 59011317 A
- JP 2008031424 A